# EUROPEAN PATENT SPECIFICATION

(11) **EP 3 755 253 B1**
(45) Date of publication and mention of the grant of the patent: **09.04.2025**
(21) Application number: 19709282.8
(22) Date of filing: 20.02.2019
(51) Int. Cl.: A61B 18/02, A61M 25/00

(54) **SYSTEMS TO ENHANCE RADIAL SPRAY FROM A CATHETER**
SYSTEME ZUR VERSTÄRKUNG DES RADIALSPRAYS AUS EINEM KATHETER
SYSTÈMES POUR AMÉLIORER UNE PULVÉRISATION RADIALE À PARTIR D'UN CATHÉTER

(30) Priority: 21.02.2018 US 201862633121 P
(43) Date of publication of application: 30.12.2020
(60) Divisional application: 25152190.2
(73) Proprietor: United States Endoscopy Group, Inc., Mentor, OH 44060 (US)
(72) Inventor: DOWNEY, George A., Arlington, Massachusetts 02476 (US); O'CONNOR, John P., Andover, Massachusetts 01810 (US); MULCAHEY, Thomas I., Bedford, Massachusetts 01730 (US); GRASSO, Daniel J., Roslindale, Massachusetts 02131 (US); FORDE, Sean, Watertown, Massachusetts 02472 (US); PALORANTA, Alexander, Cambridge, Massachusetts 02139 (US)
(74) Representative: Petraz, Gilberto Luigi
(86) International application number: PCT/US2019/018829
(87) International publication number: WO 2019/164991

(56) References cited:
- EP-A1- 2 254 637
- WO-A1-2013/062846
- WO-A2-02/07625
- US-B1- 6 179 816

## Description

### PRIORITY

This application claims the benefit of priority under 35 USC§ 119 to United States Provisional Patent Application Serial No. 62/633,121, filed February 21, 2018**.**

### FIELD

The present disclosure relates generally to the field of medical devices. In particular, the present disclosure relates to methods and devices to enhance radial spray from a catheter. Devices with flow distribution elements for a radial spray catheter, including radial cryospray catheters, are disclosed.

### BACKGROUND

Various catheters are used within different body lumens for different applications, including to deliver fluids, as a diagnostic or treatment option, to the body lumen. The fluid may be a liquid, a gas, or a mixture of both a liquid and a gas. The delivery may involve spraying the fluid on the wall of the body lumen. For purposes of delivering a catheter through an endoscope within a body lumen, in some cases, involving multiple radial apertures, the efficacy and/or efficiency of the procedure may be dependent on how uniformly the flow may be distributed among the apertures. For example, fluid may tend to flow to the endmost apertures at the distal end of a catheter more so than flowing out of more proximal apertures, creating a non-uniform distribution of flow. For example, document WO-A-2013/062846 discloses systems and methods for variable injection flow. Document US-B-6179816 discloses a catheter with uniform spray pattern along infusion length.

As an example, cryosurgery is a procedure in which diseased, damaged or otherwise undesirable tissue (collectively referred to herein as "target tissue") is treated by focal delivery of a cryogen under pressure, which may be a cryogen spray. These systems are typically referred to as cryoablation systems, cryospray systems, cryospray ablation systems, cryosurgery systems, cryosurgery spray systems and/or cryogen spray ablation systems. As typically used, "cryogen" refers to any fluid *(e.g*., gas, liquefied gas or other fluid known to one of ordinary skill in the art) with a sufficiently low boiling point *(i.e.,* below approximately -153°C) for therapeutically effective use during a cryogenic surgical procedure. Suitable cryogens may include, for example, liquid argon, liquid nitrogen and liquid helium. Pseudo-cryogens such as carbon dioxide and liquid nitrous oxide that have a boiling temperature above -153°C but still very low (e.g., -89°C for N2O) may also be used.

During operation of a cryospray system, a medical professional (*e.g*., clinician, technician, medical professional, surgeon etc.) directs a cryogen spray onto the surface of a treatment area via a cryogen delivery catheter. The medical professional may target the cryogen spray visually through a video-assisted device or endoscope, such as a bronchoscope, gastroscope, colonoscope, or ureteroscope. Cryogen spray exits the cryogen delivery catheter at a temperature ranging from 0°C to -196°C, causing the target tissue to freeze or "cryofrost."

Body lumens (e.g., the esophagus, trachea, intestines, etc.) may be treated with cryoablation via radial spray from a catheter. However, as noted above, distributing flow of cryogen mixtures through a catheter central lumen, such as liquid nitrogen and its vapor, to multiple apertures of a catheter may be difficult due to the higher density and, by extension, momentum of the liquid component of cryogen when compared to the gaseous component of cryogen in the cryogen mixture. The gaseous portions of the cryogen mixture may easily flow out of the more proximal apertures (e.g., radial apertures) while the liquid portion of the cryogen may continue to flow axially to the more distal apertures, resulting in a flow imbalance. A flow imbalance among rows of apertures may limit the uniformity and effective length of a cryogen catheter's spray volume and coverage area.

Various advantages therefore may be realized by the devices, systems and methods of the present disclosure for enhancing radial spray from catheters utilizing flow distribution elements.

### SUMMARY OF THE INVENTION

The invention is defined by the appended independent claim. Further embodiments of the invention are illustrated in the dependent claims.

### SUMMARY OF THE DISCLOSURE

The present disclosure in its various embodiments includes methods and devices to enhance radial spray from a catheter. Various embodiments may include devices for a radial spray catheter and/or a radial cryospray catheter. Various embodiments may be used with cryosurgery systems configured to enhance radial cryospray with different elements to improve flow distribution. Devices for a radial spray catheter, including radial cryospray catheters and plugs, may emit spray more efficiently and may result in more effective treatment for targeted tissue. Devices for radial cryospray catheters or other devices, or radial cryospray catheters or other devices, with flow distribution elements, may contribute to more uniform distribution of the spray and efficiently orienting the spray laterally (normal to the target) from the apertures, whereas devices without a flow distribution element may have an undesirable substantial axial component to the spray velocity or direction, or both.

The present disclosure in various embodiments includes devices and methods of use for enhanced radial spray from a catheter. Enhanced spray may be used to more efficiently delivery fluids to treatment areas to provide, among other benefits, a more productive coverage of spray at treatment sites. Various embodiments have flow distribution elements, either as a component or accessory for use with a catheter or as an integral part of spray catheters.

In one aspect of the present disclosure, a device for a radial spray catheter may include a body that may have a longitudinal axis, a proximal end, a distal end, a mid-portion extending therebetween, and an exterior radial surface. A central lumen may extend within the body along the longitudinal axis from the proximal end of the body into at least the mid-portion of the body. One or more apertures may be distributed about the exterior radial surface of the body. A flow distribution element may be in fluid communication with the central lumen and the one or more apertures. The one or more apertures may be radial openings, or slot openings, or both. The central lumen may extend through the distal end of the body and may be configured to accept a medical instrument. The central lumen may transition from a smaller diameter to a larger diameter between the proximal end of the body and the mid-portion of the body. The proximal end of the body may be configured to be mated with a distal end of the catheter. The proximal end of the body may be mated by being a continuous extension of the distal end of the catheter, bonded to the distal end of the catheter, or removably coupled to the distal end of the catheter.

In another aspect of the present disclosure, the flow distribution element may include a diffuser element that is coaxial with the central lumen and may face proximally along the longitudinal axis of the body. The diffuser element may include a cone with a cone apex that faces proximally along the longitudinal axis of the body. The flow distribution element may include a plurality of lumens fluidly connecting the central lumen with a plurality of the one or more apertures. Each lumen may extend distally within the body parallel to the longitudinal axis and may then transition along a radial wall of the body that may be perpendicular to the longitudinal axis to a corresponding aperture. The body may be ellipsoid-shaped with the major axis of the ellipsoid shape coinciding with the longitudinal axis of the body. Each lumen may extend distally within the body parallel to the longitudinal axis and may then transition along a radial wall of the body that is perpendicular to the exterior radial surface of the body to a corresponding aperture. The plurality of lumens may include discrete components that are configured to be nested together to form the body. The lumens may extend distally within the body parallel to the longitudinal axis and may then transition gradually along a curve to corresponding apertures. The central lumen may transition from the smaller diameter to the larger diameter at an angle of about 30 degrees from the longitudinal axis in the direction of the large diameter. A porous sheath or porous rings may be about the exterior radial surface of the body covering the apertures. The flow distribution element may include a plurality of independent lumens comprising elongate tubes, and each tubular lumen may be associated with an independent aperture. Each tubular lumen may have a proximal portion that extends distally within the body parallel with the central lumen and along a curve to a radial portion of the tubular lumen that may be aligned with the associated aperture. The tubular lumens may be aligned in concentric radial circles at the proximal portion. The lumens radially closer to the longitudinal axis of the body may extend farther distally at the radial portion than the lumens radially farther from the longitudinal axis of the body. The flow distribution element may include a porous body within the mid-portion of the body. The porous body may be configured to be gradually less permeable along the longitudinal axis of the body from a proximal end of the porous body to a distal end of the porous body. The porous body may be gradually more permeable from the longitudinal axis of the body in a direction toward the exterior radial surface of the body.

In another aspect of the present disclosure, the flow distribution element may include a distribution lumen that extends from the central lumen to the distal end of the body and is in fluid communication and substantially coaxial with the central lumen. The distribution lumen may have sections in the direction of the distal end along the longitudinal axis of the body that change in inner diameter and may each include at least one of the apertures. The change in inner diameter may become larger from section to section in the direction of the distal end or may become smaller from section to section in the direction of the distal end, or some combination thereof. One or more of the apertures may have a diameter that becomes larger from section to section in the direction of the distal end, or becomes smaller from section to section in the direction of the distal end, or some combination thereof. The change in inner diameter may be inversely proportional to a change in wall thickness of the body from section to section along the distribution lumen. The diameter of the exterior radial surface of the body along the distribution lumen may be constant. A section in the proximal portion of the distribution lumen may have a smaller diameter than the central lumen. The flow distribution element may have a distribution lumen that extends from the central lumen to the distal end of the body and is in fluid communication with the central lumen. The distribution lumen may include a plurality of the apertures along the longitudinal axis. A spring may be within the distribution lumen having a distal component associated with the distal end of the body and a proximal component associated with an oscillator body. The oscillator body may oscillate in the distribution lumen with flow pushing against a restoring force of the spring to distribute the flow to the apertures. The spring may be a pair of magnets with one magnet of the pair as the distal component of the spring and the other magnet of the pair as the proximal component and the oscillator body. Like poles of the magnets may face one another that act as the restoring force of the spring. The spring may have the distal component attached to the distal end of the body and the proximal component attached to the oscillator body. A volume of gas may be compressed distally behind the oscillator body that acts as the restoring force of the spring. The oscillator body may have a diffuser element having a larger diameter toward the distal end of the body and a smaller diameter of the diffuser element pointing against a direction of the flow from the proximal end of the body.

In another aspect of the present disclosure, the flow distribution element may include a distribution lumen within the body extending from the central lumen. A rod may be rotatably disposed within the distribution lumen along the longitudinal axis of the body. A turbine may be axially disposed about the rod. A multilumen member may be disposed about the rod, distal to the turbine and extending along the rod. Each lumen of the multilumen member may have an exposed radial portion that longitudinally coincides with a respective one of a plurality of radial rows of the apertures. Each lumen of the multilumen member may terminate at a substantially radial wall that is adjacent distally to the respective one of the radial rows of apertures for each lumen.

In another aspect of the present disclosure, a device for a radial spray catheter may include an elongate member having a longitudinal axis, an open proximal end, a distal end, and plurality of lumens extending therebetween in fluid communication with a flow distribution element. The flow distribution element may be disposed about the elongate member and may include a plurality of longitudinally adjacent annular chambers. Each chamber may have a proximal end, a distal end, a central lumen extending therethrough that receives the elongate member, and a plurality of radial apertures about an outer surface of the chamber. Each one of the plurality of lumens of the elongate member may be dedicated to a respective each one of the plurality of chambers and may have at least one dedicated supply aperture in fluid communication therewith. Each lumen of the elongate member may terminate at the at least one dedicated supply aperture associated with its respective annular chamber. The elongate member may be mated to a distal end of a catheter by being a continuous extension of the distal end of the catheter, bonded to the distal end of the catheter, or removably coupled to the distal end of the catheter.

In another aspect of the present disclosure, a device for a radial spray catheter may include an elongate body configured to be inserted into a distal end opening of a catheter. The body may have a longitudinal axis, a proximal end, and a distal end. A flow distribution element may extend at least partially between the proximal and distal end of the body and may include a backstop at the distal end of the body. The flow distribution element may include a plurality of fins extending radially from the longitudinal axis of the elongate body. The fins may be configured to engage an inner surface of the catheter. The backstop may have a surface perpendicular to and facing the distal end opening of the catheter when the elongate body is inserted into the catheter. The surface may be longitudinally offset from the distal end opening of the catheter in a distal direction forming a radial aperture around the opening. The flow distribution element may include a plurality of fins extending along the elongate body and arranged in a helical pattern that widens radially further from the longitudinal axis of the elongate body as the fins extend to the backstop. The backstop may be configured to engage an inner surface of the catheter. The backstop may have a concave surface facing the distal end opening of the catheter. The flow distribution element may include a diffuser element extending proximally from the concave surface against a direction of flow from the opening of the catheter. The concave surface may be offset from the distal end of the elongate body, creating a radial aperture around the opening.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 illustrates an isometric view of a cryosurgery system in accordance with an embodiment of the present disclosure.
FIG. 2 illustrates a radial spray tip of a catheter in accordance with an embodiment of the present disclosure.
FIG. 3A illustrates a left view of a device with a flow distribution element including lumens in accordance with an embodiment of the present disclosure.
FIG. 3B illustrates a cross-sectional view of the embodiment in FIG. 3A.
FIG. 3C illustrates a left view of a device with a flow distribution element including lumens in accordance with an embodiment of the present disclosure.
FIG. 4A illustrates a left view of a device with a flow distribution element including lumens in accordance with an embodiment of the present disclosure.
FIG. 4B illustrates a cross-sectional view of the embodiment of FIG. 4A.
FIG. 4C illustrates a left view of a device with a flow distribution element including lumens and a sheath in accordance with an embodiment of the present disclosure.
FIG. 4D illustrates a left view of a device with a flow distribution element including lumens and a scalloped outer surface in accordance with an embodiment of the present disclosure.
FIG. 5A illustrates a left, quarter-cross-sectional view of a device with a flow distribution element including a plurality of tubular lumens in accordance with an embodiment of the present disclosure.
FIG. 5B illustrates a cross-sectional view the embodiment of FIG. 5A.
FIG. 6 illustrates a cross-sectional view of a device with a flow distribution element including a porous body in accordance with an embodiment of the present disclosure.
FIG. 7A illustrates a perspective, cross-sectional view of a device with a flow distribution element including a distribution lumen that has a change in diameter in accordance with an embodiment of the present disclosure.
FIG. 7B illustrates a perspective, cross-sectional view of a device with a flow distribution element including a distribution lumen that has a change in diameter in accordance with an embodiment of the present disclosure.
FIG. 8A illustrates a left view of a device with a flow distribution element including a distribution lumen with an oscillator body attached to a spring in a first position in accordance with an embodiment of the present disclosure.
FIG. 8B illustrates a left, cross-sectional view of the embodiment of FIG. 8A with the spring in a second position.
FIG. 9A illustrates perspective view of a device with a flow distribution element including a distribution lumen with a rotatable turbine in accordance with an embodiment of the present disclosure.
FIG. 9B and 9C illustrate an axial, cross-sectional view of the embodiment of FIG. 9A in a first and second position, respectively.
FIG. 10A illustrates a perspective view of a device with a flow distribution element including a distribution lumen with a plurality of annular chambers in accordance with an embodiment of the present disclosure.
FIG. 10B is a substantially rear view of the embodiment of FIG. 10A.
FIG. 10C is a perspective view of the distribution member of FIGS 10A and 10B.
FIG. 10D is a perspective view of the annular chamber of FIGS 10A and 10B.
FIG. 11 is a perspective view of a device with a flow distribution element including an insert for a catheter in accordance with an embodiment of the present disclosure.
FIG. 12A is a perspective view of a device with a flow distribution element including an insert for a catheter in accordance with an embodiment of the present disclosure.
FIG. 12B is a left view of the embodiment of FIG. 12A.
FIG. 13 is a right, cross-sectional view of a device with a flow distribution element including a backstop having a concave surface in accordance with an embodiment the present disclosure.

### DETAILED DESCRIPTION

The present disclosure is not limited to the particular embodiments described. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs. Although embodiments of the present disclosure are described with specific reference to radial cryospray systems for use within the upper and lower GI tracts and respiratory system, it should be appreciated that such systems and methods may be used in a variety of other body passageways, organs and/or cavities, such as the vascular system, urogenital system, lymphatic system, neurological system and the like.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

As used herein, the conjunction "and" includes each of the structures, components, portions, or the like, which are so conjoined, unless the context clearly indicates otherwise, and the conjunction "or" includes one or the others of the structures, components, portions, or the like, which are so conjoined, singly and in any combination and number, unless the context clearly indicates otherwise.

As used herein, the term "distal" refers to the end farthest away from the medical professional when introducing a device into a patient, while the term "proximal" refers to the end closest to the medical professional when introducing a device into a patient. As used herein, "diameter" refers to the distance of a straight line extending between two points and does not necessarily indicate a particular shape.

The present disclosure generally provides methods and devices to enhance radial spray from a catheter. Various embodiments may include devices to enhance spray for a radial spray catheter and/or a radial cryospray catheter.

For example, various embodiments of devices for enhanced spray, described here or otherwise, within the scope of the present disclosure, may be used with cryosurgery systems configured with flow distribution elements to improve uniformity of flow distribution. Exemplary cryosurgery systems in which the present disclosure may be implemented include, but are not limited to, those systems described in U.S. Patent Nos. 9,301,796 and 9,144,449, and U.S. Patent Application Serial Nos. 14/012,320 and 14/869,814, features and advantages of distributing fluid through flow distribution elements may be realized with a lumen running the length of the element that accommodates a medical instrument extending therethrough. Such elements may be implemented with features throughout the disclosure of co-owned United States Provisional Patent Application having Attorney Docket number 8177.0040, filed concurrently herewith.

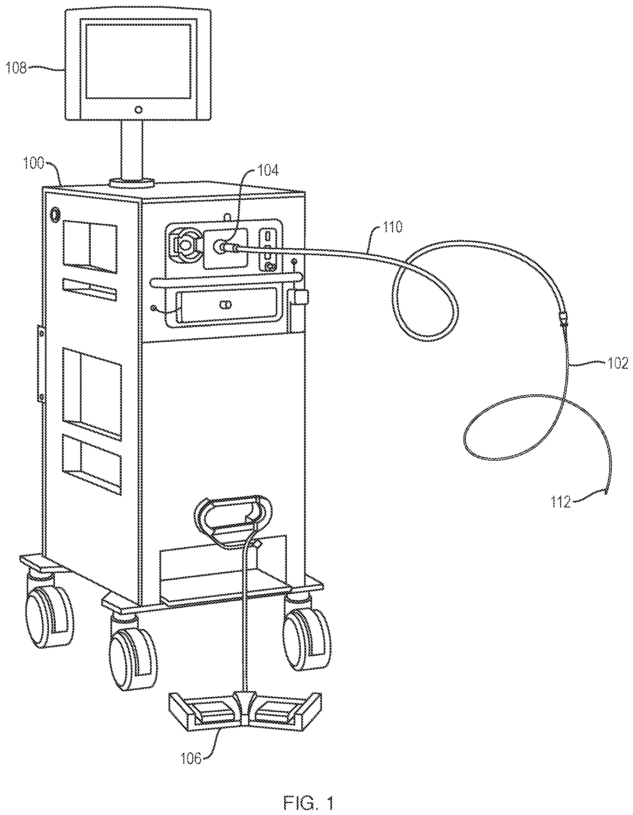

In one embodiment of a cyrospray delivery system configured for devices to enhance cryospray from a catheter, as illustrated in FIG. 1, a catheter 102 is connected to a cryotherapy console 100 at a catheter interface 104. The catheter 102 may be used with an endoscope for delivery into a patient. An image received at the lens on the distal end of the endoscope may be transferred to a monitoring camera which sends video signals via a cable to the monitor 108, where the procedure can be visualized. Built-in software and controls in the console allows the medical professional to control delivery of cryogen from the tank through the catheter 102 via a foot petal 106. The catheter 102 may have an insulated portion 110 and a distal end 112.

As an example of the fluid mechanics of cryospray formation and supply, with reference to the system illustrated in FIG. 1, as cryogen (e.g., liquid nitrogen) travels from the tank to the proximal end of cryogen delivery catheter 102, the liquid warms and starts to boil, resulting in cool gas emerging from the distal end 112 of catheter 102. The amount of boiling in the catheter 102 depends on the mass, surface area, and thermal capacity of catheter 102. When the liquid nitrogen undergoes phase change from liquid to gaseous nitrogen, additional pressure is created throughout the length of catheter 102. This is especially true at a solenoid/catheter junction, where the diameter of the supply tube to the lumen of catheter 102 decreases, e.g., from approximately 6.35 mm to approximately 1,78mm (0.25 inches to approximately 0.070 inches), respectively. The lumen of the catheter 102 may have a diameter, e.g., ranging between 0.76 mm and 2.92 mm (0.030 and 0.115 inches) In other embodiments, the lumen may have another range of diameter depending on what is suitable for a particular application. In an alternate embodiment, gas boiling inside the catheter 102 may be reduced even greater by the use of insulating materials such as PTFE, FEP, Pebax, and the like, or by surrounding the catheter with a substantially evacuated lumen to help reduce the rate of heat transfer.

With further reference to FIG. 1, as an example, the catheter 102 is connected to a console 100. The console 100 contains the tank that supplies the cryogen. The console 100 may include precooling and defrost features. The console 100 and/or catheter 102 include valving and plumbing to deliver the cryogen under pressure, e.g., delivering low pressure to the distal tip 112 of the catheter 102. There may be sensors within the console 100 and/or the catheter 102 to measure the temperature of the cryogen and/or the tissue. There may be a feedback loop for metered control of cryogen dosing. A pedal 106 may be used to control the cryogen delivery, or the cryogen delivery may be timed for a predetermined dosage. The distal tip 112 may be openended and/or include radial apertures. The console 100 may include software and/or hardware with safety features. The console 100 may include an interactive user interface. The console 100 may include control settings for a cryospray therapy procedure. The console 100 may include cryospray procedure profiles for pre-determined delivering of cryospray.

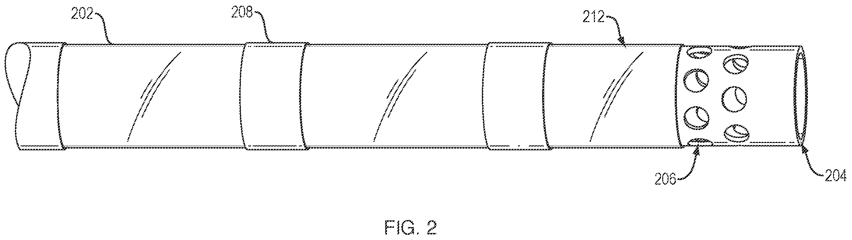

With reference to FIG. 2, an exemplary cyrospray catheter in accordance with an embodiment of the present disclosure is illustrated. The catheter 202 is placed within a patient such that the distal end 212 is in proximity to the target tissue. A medical professional may visualize placement of the distal end 212 of the catheter 202 via a camera in an endoscope and/or through fluoroscopy. The marking bands 208 may be visualized using the camera and/or may be radiopaque for visualization with a fluoroscope. With the distal end 212 in position, the medical professional may introduce cryogen into the catheter 202. When the cryogen reaches the distal end 212 of catheter 202 it exits the distal tip 204 and/or the radial apertures 206 as a cyrospray towards the target tissue. The exemplary catheter 202 is shown in FIG. 2 with an open end with an open distal face to produce spray in a distal direction at the distal tip 204 and with radial apertures 206 to produce spray in a radial direction. Alternative exemplary catheters may have only a distal tip and open distal face without having any radial apertures, or may have only radial apertures without having a distal tip with an open distal face.

With the system of FIG. 1 and/or catheter of FIG. 2, for example, freezing of fluids on the target tissue and/or freezing of the target tissue is apparent to the medical professional by the acquisition of a white color by the target tissue. The white color, resulting from surface frost, indicates the onset of mucosal or other tissue freezing sufficient to initiate destruction of the diseased or abnormal tissue. The operator may use a system timer to freeze for a specified duration once initial freeze is achieved in order to control the depth of injury. The delivery of cyrogen may be metered and controlled via a feedback loop that monitors readings from one or more temperature sensors on the catheter shaft. The medical professional may observe the degree of freezing and stop the spray as soon as the surface achieves the desired whiteness of color. The operator may monitor the target tissue to determine when freeze has occurred via the camera integrated into the endoscope. The operator may manipulate the catheter to freeze the target tissue. Once the operation is complete, the catheter, endoscope, and any other instruments, such as a cryodecompression tube for the evacuation by passive or active venting of fluids from the patient (e.g., cryospray gases), are withdrawn from the patient.

The delivery of a multiphase flow of cryogen through the catheter 202 leads to the radial apertures 206 and/or distal tip 204 for cryospray to exit the catheter. Cryogens may partially boil as they travel down the catheter 202 and the resulting mixture may be released out of the exit points at the distal end 212 of the catheter 202. The radial apertures 206 in the distal end 212 of the catheter 202 are meant to emit cryospray onto the inner wall of tissue in a body lumen.

When cryospray exits the distal end 212 of the catheter 202 through the radial holes 206, it does so typically in an orthogonal direction or angle from the catheter 202 (*i.e.,* along an axis transverse to the longitudinal axis of the catheter). Increasing the ratio of the width of radial holes to the diameter of these apertures may improve spray orthogonality, which may increase cooling efficiency.

As noted above, distributing flow of cryogen mixtures (e.g., liquid and gas, such as liquid nitrogen and its vapor) through a catheter central lumen to multiple apertures may be difficult due to the higher momentum of the liquid cryogen when compared to the gaseous cryogen in the cryogen mixture. The gaseous portions of the cryogen mixture may easily flow out of the more proximal apertures (e.g., radial apertures), while the liquid portion of the cryogen may continue to flow axially to the distal end of the catheter, resulting in a flow imbalance. This flow imbalance among rows of apertures may limit the uniformity and effective length of a cryogen spray volume and coverage area. The momentum of fluid flow within a catheter and/or catheter tip may also be difficult to direct efficiently among a plurality of apertures arranged at different points longitudinally and radially.

Distal momentum of fluid traveling within a catheter may still exist when sprayed out of the apertures. This distal momentum may progress fluids distally past the spray site, which may be undesirable and may result in patient harms such as distention or perforation of organs.

Various embodiments of devices with different configurations of flow distribution elements may improve the uniformity of flow distribution across one or more radial apertures and increase the efficiency and efficacy of radial spray. Some of the embodiments may have a body with a longitudinal axis, a proximal end, a distal end, a mid-portion extending therebetween, and an exterior radial surface. A central lumen may extend within the body along the longitudinal axis from the proximal end of the body into at least the mid-portion of the body. One or more apertures may be distributed about the exterior radial surface of the body. The apertures may be radial openings, or slot openings, or both. The central lumen may include a lumen of a catheter, or include a portion of a lumen of a catheter, or include a proximal section of a body that does not include the lumen of a catheter. The central lumen may extend through the distal end of the body, e.g., to allow passage of instruments through the central lumen. The central lumen may transition from a smaller diameter to a larger diameter between the proximal end of the body and the mid-portion of the body. This transition may be at an angle, e.g., of about 25 to about 30 degrees from a longitudinal axis of the body in the direction of the large diameter. A flow distribution element may be in fluid communication with the central lumen and the one or more apertures. The flow distribution element may extend from the central lumen to a plurality of apertures about the body. The flow distribution element may help the flow of cryogen fluids from the central lumen to transition through the flow distribution element and out of the apertures more efficiently and with less flow imbalance. The various embodiments of devices with a flow distribution element may be mated with an end of a catheter, e.g., inserted into, bonded with, removably coupled to, or integrated into the end of a catheter as a continuous extension or unibody of the catheter. Various embodiments may include apertures that vary in diameter and/or in depth along the length of the body of the device. Lumens leading to apertures may vary in diameter and shape. Such variances may increase or decrease flow to particular apertures such that a net uniform or other desired application of spray for all of the apertures results.

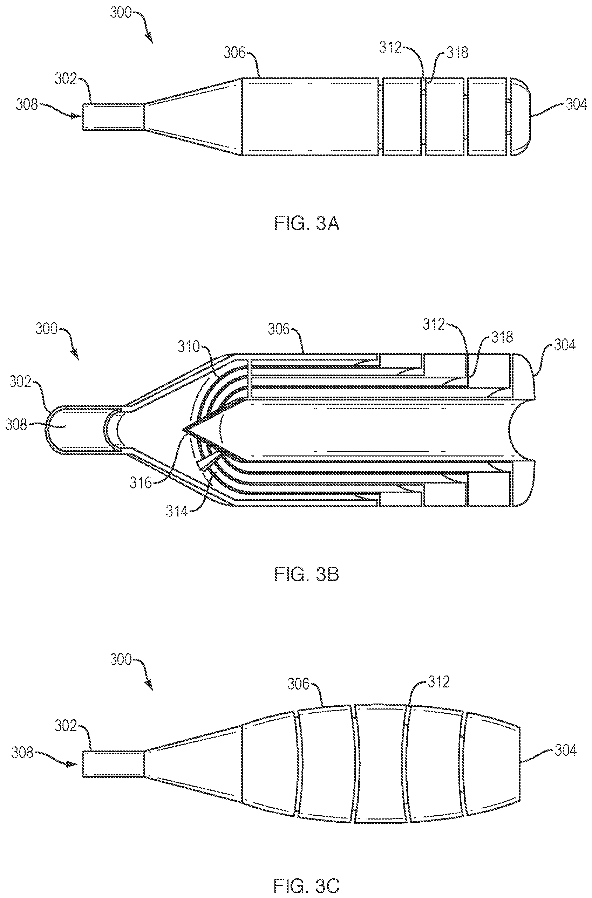

With reference to FIGS. 3A-3C, an embodiment of a device with a flow distribution element 310 includes lumens 314 fluidly connecting a central lumen 308 to a plurality of apertures 312 about a body 300. The flow distribution element 310 includes a diffuser element, e.g., cone 316, at a proximal end of the flow distribution element 310 with an apex of the cone 316 that is axial with the central lumen 308. The apex of the cone 316 is directed toward the central lumen 308 *(i.e.,* in a proximal direction). A flow of cryogen through the central lumen 308 from the proximal end 302 is expanded conically to a larger diameter, because of the cone 316, to enhance uniform distribution to a proximal end of the lumens 314. The angle of the cone 316 (as well as the angle of the wall of the body as the central lumen expands to a mid-portion of the body) may be an angle characteristic of a saturated multiphase mixture expansion, e.g., about 25° to about 30° for liquid nitrogen from a longitudinal axis of the body. The flow distribution element is then separated by multiple lumens 314. Each lumen 314 corresponds to a separate aperture 312 (e.g., a row of radial apertures, a radial slot, or a ring). Each lumen 314 extends distally within the body 300 parallel to the longitudinal axis and then transitions to an aperture 312 along a radial wall 318 of the body 300 that is perpendicular to the longitudinal axis to a corresponding aperture 312. The plurality of lumens 314 may include discrete components that are configured to be nested together to form the body 300. When the flow from a lumen 314 reaches the radial wall 318, the flow momentum changes from a substantially distal, axial direction to a substantially radial direction, towards the aperture 312. While the body 300 may be substantially cylindrical as in FIGS. 3A and 3B, the body 300 may take on other shapes, such as an ellipsoid as in FIG. 3C. An ellipsoid or other shape of body may be configured with different paths for the lumens to the apertures, e.g., other than perpendicular to the longitudinal axis of the body 300, in order to direct spray in a multitude of directions, such as a direction normal to a curved outer surface of the body 300, such as an angle from an axis that is perpendicular to the longitudinal axis of the body where the angle may be 5°, 10°, 15°, or the like (e.g., FIG. 3C). For example, the body 300 depicted in FIG. 3C may be ellipsoid-shaped with the major axis of the ellipsoid shape coinciding with the longitudinal axis of the body 300. Each lumen 314 may extend distally within the body 300 parallel to the longitudinal axis and then transition along a radial wall of the body 300 that is perpendicular to the exterior radial surface of the body 300 to a corresponding aperture 312. Exterior radial surfaces may correspond to an anatomy that is to be treated. For example, the spray from each aperture may be oriented normal to the surface of the target tissue. Anatomies such as a hiatal hernia may be treated with such an elliptical exterior radial surface of a body. A flow distribution element 310 may include different configurations of a diffuser element that are coaxial with the central lumen 308 and face proximally along the longitudinal axis toward the proximal end 302 of the body 300 to distribute flow, such as the cone 316 described above, or a device may not include the cone 316 or other diffuser element such that flow may pass through a central lumen 308 in a distal direction out of the body 300.

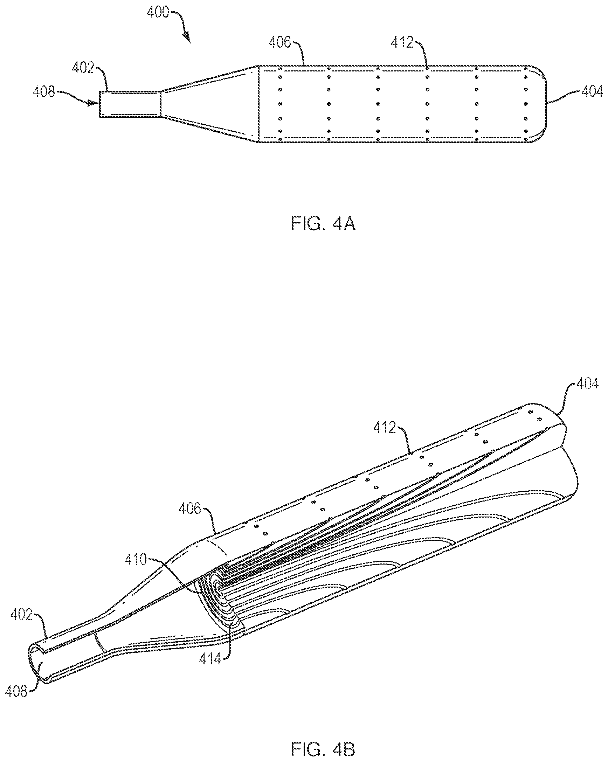

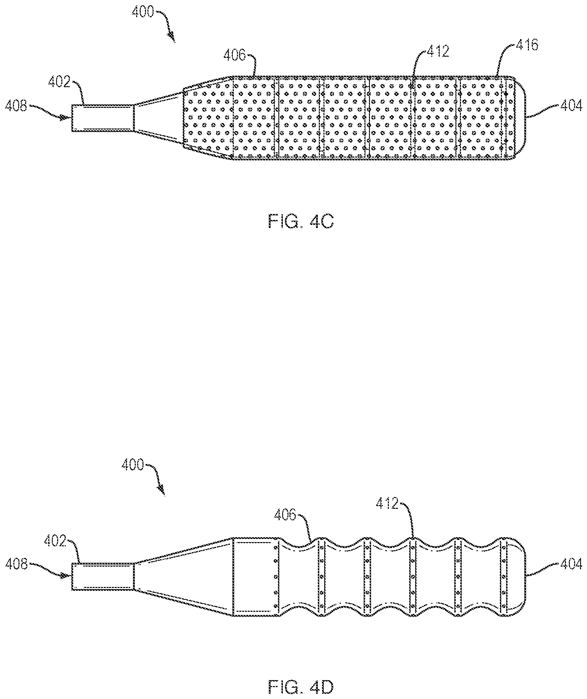

With reference to FIGS. 4A-4D, an embodiment of a device with a flow distribution element 410 includes lumens 414 extending from the central lumen 408 to a plurality of apertures 412 about the body 400. A flow of cryogen through the central lumen 408 expands substantially conically to a larger diameter because of the transition from the smaller diameter of the central lumen 408 compared to the larger diameter of the mid-portion of the portion corresponding to outermost lumen 410. The angle of the transition may be an angle characteristic of a saturated multiphase mixture expansion, e.g., about 25° to about 30° for liquid nitrogen. The flow is then distributed through the multiple lumens 410. Each lumen 410 corresponds to a separate row of apertures 412 (e.g., radial apertures or radial slots or rings). As shown in FIG. 4B. the lumens 410 extend distally within the body 400 parallel to the longitudinal axis and then transition gradually along a curve to corresponding apertures 412, but the path may be as desired including as described above with respect to FIGS. 3A-3C. With a gradual curve, flow within an annular channel 410 may transition from an axial direction to a radial direction along the length of the lumen 410 with a longer radius of curvature and greater angle, than a 90-degree angle turn. Referring to FIG. 4C, an embodiment of a porous sheath 416 is depicted. The sheath may be disposed about the exterior surface of the body 400 and cover the apertures 412. The porous sheath 416 may comprise one or more porous rings and each ring may correspond to or cover a row of apertures 412. A porous sheath, such as sheath 416, may be placed over any embodiment of the present disclosure in order to help disperse the cryogen flow from an aperture into a uniform plume. A sheath may be fabricated in a multitude of ways such as into a woven mesh or with sintered particles having pore sizes chosen to optimally disperse the spray plume. The sheath may diffuse the spray from the apertures 412 to create a more continuous spray pattern when compared to an array of apertures 412 without a sheath. Particles of the spray emitted from the apertures 412 may be sintered by the sheath 416, resulting in a substantially randomized and substantially uniform mist of spray. While the body 400 may be substantially cylindrical as in FIGS. 4A-4C, the body 400 may take on other shapes, such as a body 400 with a scalloped exterior radial surface between the apertures 412, as in FIG. 4D. The scalloped shape decreases the amount of material necessary to manufacture the embodiment. The apertures 412 of FIGS. 4A and 4B may instead comprise of rings such as that illustrated in 4C, but without the sheath 416.

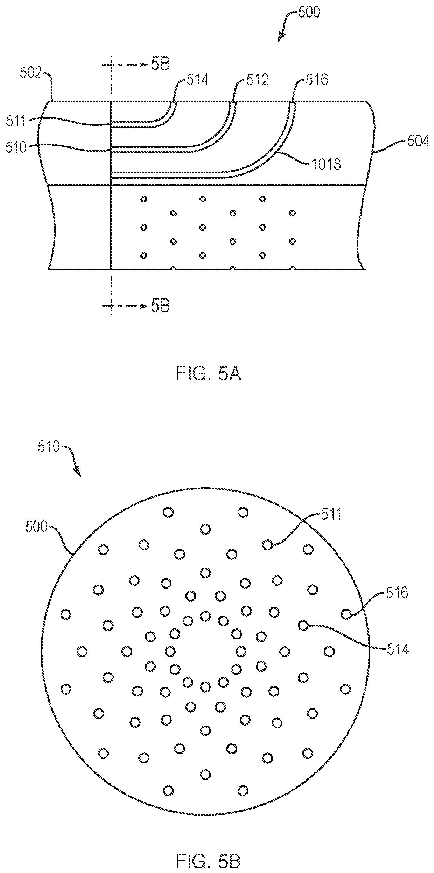

With reference to FIGS. 5A and 5B, an embodiment of a device with a flow distribution element 510 includes independent lumens 511 comprising elongate tubes extending from the central lumen 508 to a plurality of apertures 512 about the body 500. Each tubular lumen 511 is associated with an independent aperture 512. Each tubular lumen 511 has a proximal portion that extends distally within the body 500 parallel with the central lumen 508 and along a curve to a radial portion of the tubular lumen 511 that is aligned with the associated aperture 512. A flow of cryogen through the central lumen of a catheter may be expanded to a larger diameter of the flow distribution element 510. The flow is then distributed through the tubular lumens 511. The tubular lumens 511 gradually extend to a radial portion that is radial with the apertures 512. Various angles of curvature, including an angle normal to the longitudinal axis, as above, may be chosen as desired for particular applications. Each tubular lumen 511 in FIG. 5A transitions to an aperture 512 gradually in an arc along the length of each tubular lumen 511. Flow within the tubular lumens 511 smoothly transitions from an axial direction to a radial direction throughout the length of the tubular lumen 511. The tubular lumens 511 are aligned in concentric radial circles at the proximal portion of the tubular lumens 511. Tubular lumens 511 that are closer 514 to the longitudinal axis of the body 500 extend farther in a distal direction at the radial portion than the tubular lumens 511 that are radially farther 516 from the longitudinal axis of the body 500.

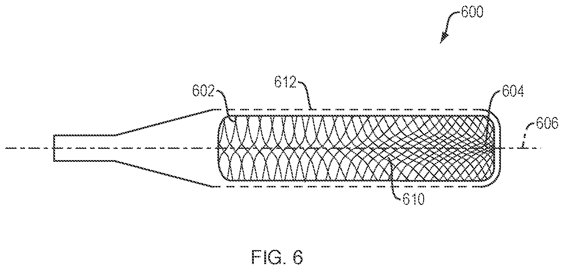

With reference to FIG. 6, an embodiment of a device with a flow distribution element includes a porous body 610 within a mid-portion of the body 600. The porous body 610 gradually becomes less permeable from a proximal end 602 to a distal end 604 along a longitudinal axis of the body. The porous body 610 also gradually becomes more permeable in a direction from the longitudinal axis 606 toward the exterior radial surface of the body 600 (*i.e.*, towards the apertures 612). The porous body 610 may be anisotropic in other directions along the porous body 610 to accommodate desirable flow and distribution properties. A porous body 610 with higher radial permeability relative to the axial permeability creates a flow pathway that will travel in a primarily radial path toward the apertures 612. A porous body, such as the body 610, may be fabricated in a multitude of configurations such as multiple stacks of woven wire screens with increasing or decreasing density. Stacked screens inherently have anisotropic flow resistances due to the geometric difference between a flow path through the screen in a normal direction and a flow path longitudinally through the screen. The porous body 610 may be designed such that flow impedance increases distally with each axial row of apertures 612 such that flow is evenly distributed among the rows (e.g., by radially uniform change in permeability).

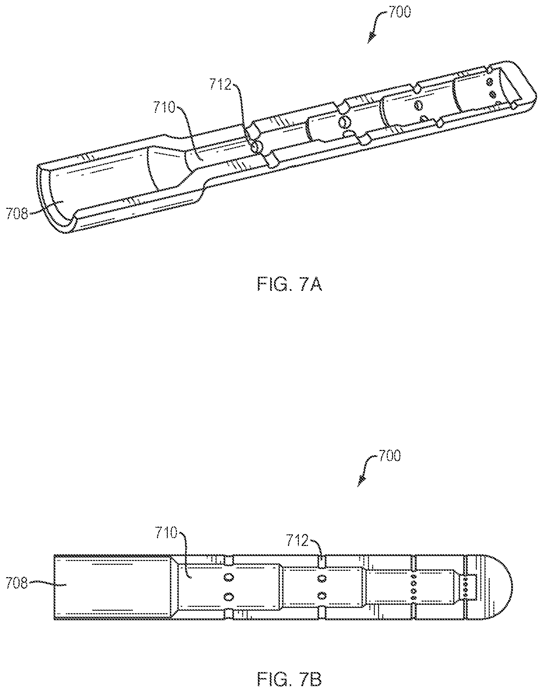

With reference to FIGS. 7A and 7B, an embodiment of a device with a flow distribution element includes a distribution lumen 710 that extends from the central lumen 708 to the distal end of the body 700 and is in fluid communication with and substantially coaxial with the central lumen 708. A plurality of apertures 712 about the body 700 are also in fluid communication with the central lumen 708. The distribution lumen 710 has sections in the direction of the distal end along the longitudinal axis of the body 700 that change in inner diameter and each include at least one of the apertures 712. Each row of apertures 712, as radial holes, diminish in diameter in a distal direction along the body 700. Because cryogen flow may be typically distributed unevenly along the length of a body *(i.e.,* more flow exits from the apertures 712 in the distal portion of a device when compared to apertures 712 in the proximal portion), larger diameter apertures 712 in the proximal portion of the device allows for a lessresistive exit pathway for the flow than that of the smaller diameter apertures 712 at the distal portion. A flow of cryogen through the central lumen 708 of this embodiment is constricted from the larger diameter of the central lumen 708 to the smaller diameter of the distribution lumen 710. The distribution lumen 710 extends in a distal direction and changes in diameter after each row of apertures 712 in the distal direction. In FIG. 7A, the change in diameter of the distribution lumen 710 is larger in the direction of the distal end. In FIG. 7B, the change in diameter of the distribution lumen 710 is smaller in the direction of the distal end. Some embodiments, e.g., FIG. 7A, may have a section in the proximal portion of the distribution lumen (e.g., 710) that has a smaller diameter than the central lumen (e.g., 708). Some embodiments may have a combination of changing diameters of sections of a distribution lumen along the body 700. The distribution lumen 710 may change in diameter in abrupt steps, or by gradual transitioning or tapering. A wall thickness about the distribution lumen 710 in FIG. 7A is reduced in a distal direction along the body 700, which relates to the depth of the apertures 712. The deeper apertures 712 in the proximal portion have a higher residence time for the flow as it exits the apertures 712 than that of the shallower apertures in the distal portion of the body 700. As such, similar flow distribution may be achieved if the change in the inner diameter of the distribution lumen is inversely proportional to a change in wall thickness of the body from section to section along the distribution lumen, such as depicted in FIGS 7A-7B. In such cases, the diameter of the exterior radial surface of the body along the distribution lumen may be kept constant. The varying diameter of the distribution lumens 710 affects the amount of fluid delivered from a flow of fluid to each aperture 712.

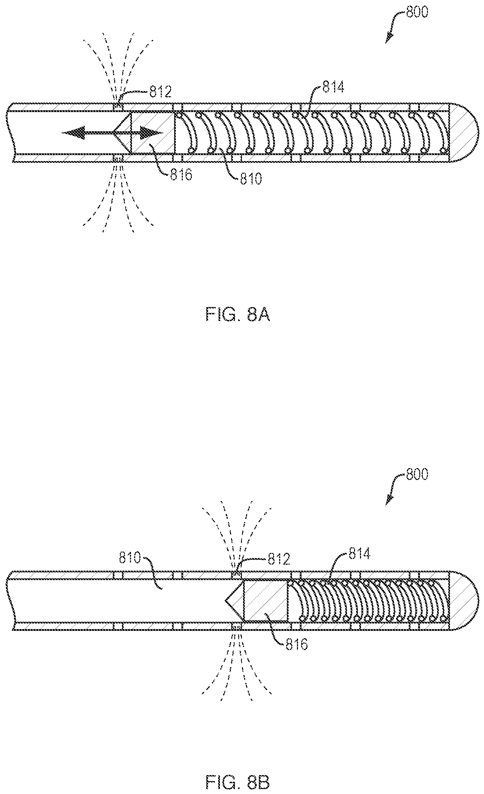

With reference to FIGS. 8A and 8B, an embodiment of a device with a flow distribution element includes a distribution lumen 810 that extends from a central lumen to the distal end of the body 800 and is in fluid communication with the central lumen. The distribution lumen 810 includes a plurality of the apertures 812 along the longitudinal axis. A spring 814 within the distribution lumen 810 has a distal end attached to the distal end of the body 800 and a proximal end. An oscillator body 816 is attached to the proximal end of the spring 814, whereby the oscillator body 816 oscillates in the distribution lumen 810 with flow pushing against a restoring force of the spring 814 to more evenly distribute the flow to the apertures 812. The oscillator body 816 has a conical diffuser element pointing against a direction of the flow from the proximal end of the body 800. A flow of cryogen through the distribution lumen 810 of this embodiment impacts the oscillator body 816 and is expanded substantially conically to a larger diameter, because of the shape of the proximal side of the oscillator body 816. The diffuser element of the oscillator 816 body may take on other shapes such as an inverted cone, a dome, an inverted dome, a flat surface, any shape with an increasing diameter, a shape with an exponentially increasing diameter, and the like. The flow is directed to the apertures 812 that are closest to the oscillator body 816. The flow of fluid approaches the oscillator body 816 in an axial direction and with axial force. This axial force translates the oscillator body 816 in a distal direction, compressing the spring 814. As the oscillator body 816 translates further distally within the distribution lumen 810, it will pass by other rows of apertures 812 that the oscillator body 816 will direct the cryogen flow to. The pulsatile nature of the flow of fluid will vary the force applied to the oscillator body 816 and the spring 814, causing the oscillator body 816 to oscillate distally and proximally, distributing the flow among the apertures 812 as the apertures 812 are uncovered. A spring and damper are designed for the flow distribution element such that a stroke that translates the oscillator body 816 axially will spend an equal amount of time passing each row of apertures 812. The time-averaged flow through each row of apertures 812 is equal, causing a uniform application of spray while reducing the instantaneous flow rate of fluid within the device and decreasing gas egression requirements. This embodiment does not supply all rows of apertures 812 evenly at the same time, and so the most-distal apertures 812 do not receive an imbalanced amount of flow compared to other apertures 812. The spring 814 may be attached within the distribution lumen 810 at a distal end or a proximal end. The spring 814 has a distal component associated with the distal end of the body 800 and a proximal component associated with the oscillator body 816. The spring 814 may be a pair of magnets with one magnet of the pair as the distal component of the spring 814 and the other magnet of the pair as the proximal component and the oscillatory body 816. Like poles of the magnets may face one another that act as the restoring force of the spring 814. The spring 814 may have the distal component attached to the distal end of the body 800, the proximal component attached to the oscillator body 816, and a volume of gas compressed distally behind the oscillator body 816 may act as the restoring force of the spring 814. The oscillatory body 816 may be disposed onto hydrodynamic bearings that may reduce friction.

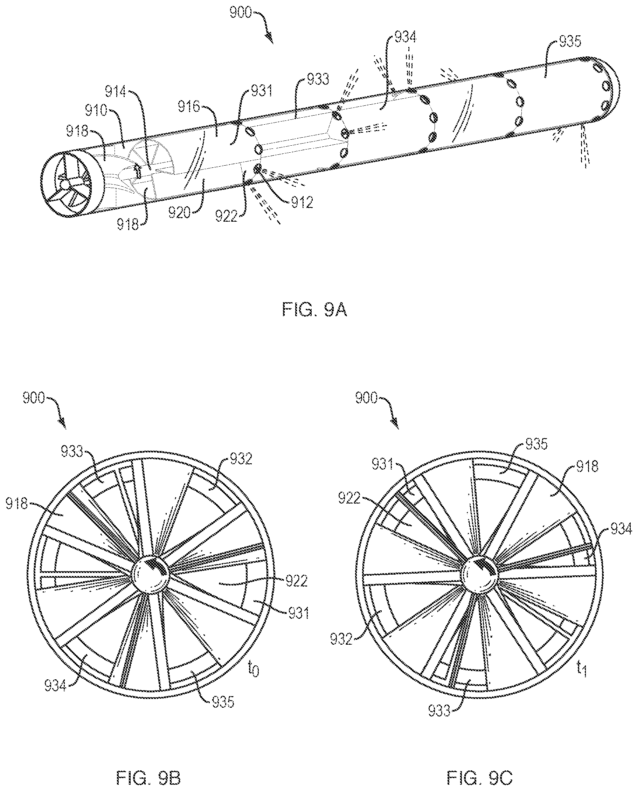

With reference to FIGS. 9A-9C, an embodiment of a device with a flow distribution element includes a distribution lumen 910 within the body 900 extending from a central lumen. A rod 914 is rotatably disposed within the distribution lumen 910 and extends along a longitudinal axis of the body 900. A turbine 918 is axially disposed about the rod 914. A flow of fluid into the distribution lumen 910 will rotate the turbine 918 and the rod 914. At least one multilumen member 916 is disposed about the rod 914 and distal to the turbine 918. The flow of fluid is divided into radial segments that make up the lumens 920 of the first multilumen member 931. At least one lumen 920 of each of the multilumen members 916 has an exposed radial portion that longitudinally coincides with one of the plurality of rows of radial apertures 912. Each lumen 920 of the multilumen member(s) 931 terminates in a substantially radial wall 922 adjacent to one of the rows of apertures 912 in a distal direction. The substantially radial wall 922 terminates the lumen 920 of the multilumen member 916 that has the exposed radial portion associated with a specific row of apertures 912. A portion of the flow of fluid that enters the lumen 920 with an exposed radial portion will collide with the substantially radial wall 922. The flow will be diverted from a substantially axial direction to a substantially radial direction out of the apertures 912 that are in fluid communication with the lumen 920 with an exposed radial portion. The remainder of the flow of fluid will travel distally through the remaining lumens 920 of the first multilumen member 931. These lumens 920 are open at a distal end of the first multilumen member 931 for the flow of fluid to reach subsequent multilumen members 916 in the distal direction. Flow from the lumens 920 that do not terminate in the substantially radial wall 922 of the first multilumen member 931 will reach the second multilumen member 932. Flow from one multilumen member 916 distally to the next will subsequently lose flow from one of the lumens 920 as flow continues from the first multilumen member 931, to the second multilumen member 932, to the third multilumen member 933, to the fourth multilumen member 934, and to the fifth multilumen member 935. Each multilumen member 916 has one substantially radial wall 922 that is rotationally offset about the rod 914 from the substantially radial wall 922 of other multilumen members 916. In this way, each of the radially exposed lumens 920 of the multilumen members 916 together form a spray pattern of about 360° about the rod 914 and through the apertures 912. This 360° spray pattern coverage is distributed through each multilumen such that each multilumen sprays in an angular arc of about 360/n where "n" is the number of multilumen members 916 (e.g., each multilumen member in FIG. 9A sprays in an angular arc of about 72°). The multilumen members 916 are attached to the rod 914 such that the turbine 918, rod 914, and multilumen members 916 rotate together within the distribution lumen 910. For example, FIG. 9B illustrates the angular position of the rotatable turbine 918, rod 914, and multilumen members 916 at time t₀. FIG. 9C illustrates the angular position of the rotatable turbine 918, rod 914, and multilumen members 916 at another instant of time t₁. At both t₀ and t₁, there is an angular spray coverage of about 360°, however, the axial depth of the spray coverage varies at each substantially radial wall 922 of each of the multilumen members 931, 932, 933, 934, and 935. The axial distance between the rows of apertures 912 and the length of the multilumen members 916 determine the amount of area covered by the fluid spray. This embodiment may create a large spray area without increasing the total flowrate necessary since only some of the apertures 912 are active at any given instant.

Referring to FIGS. 8A through 9C, embodiments of a device for enhanced spray according to the present disclosure may increase body lumen coverage of cryospray, without increasing the flowrate of fluid. These embodiments and the other embodiments of flow distribution elements described above, may allow for more efficient and accurate control of the rate and volume of fluid necessary to provide for treatment of the body lumen, while avoiding excess gas build up that may result in distension or other harmful effects. Exposing only a portion of the apertures to the fluid within the flow distribution element at a time, may allow for a lower flow rate of fluid when compared to an embodiment exposing all of the apertures to the flow of fluid throughout a treatment procedure. Even though only a portion of the apertures are exposed at one time, complete coverage of cryospray along all of the apertures may be maintained. A lower flow rate may result in less distal progression of the cryospray, which may be useful in certain anatomies and/or if maintaining a uniform gas egression rate is difficult.

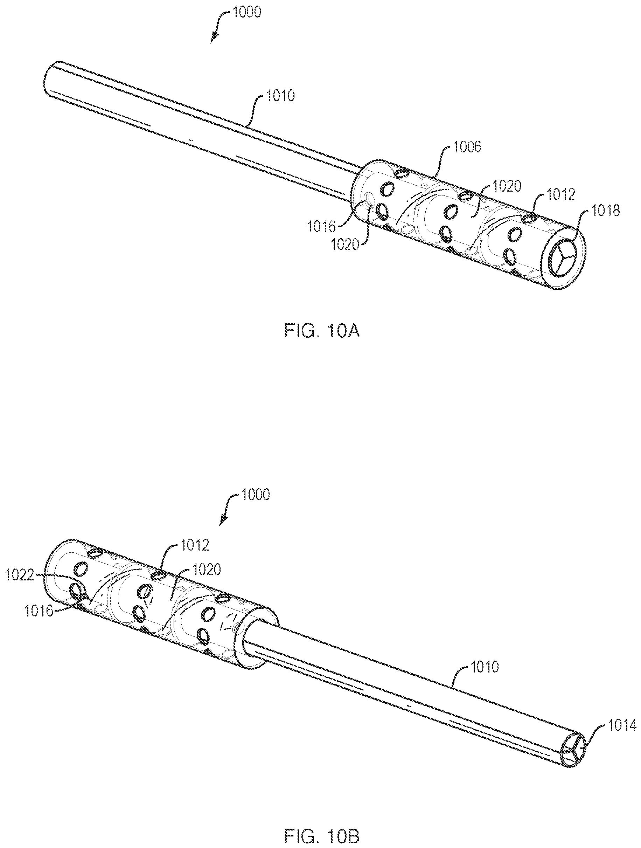

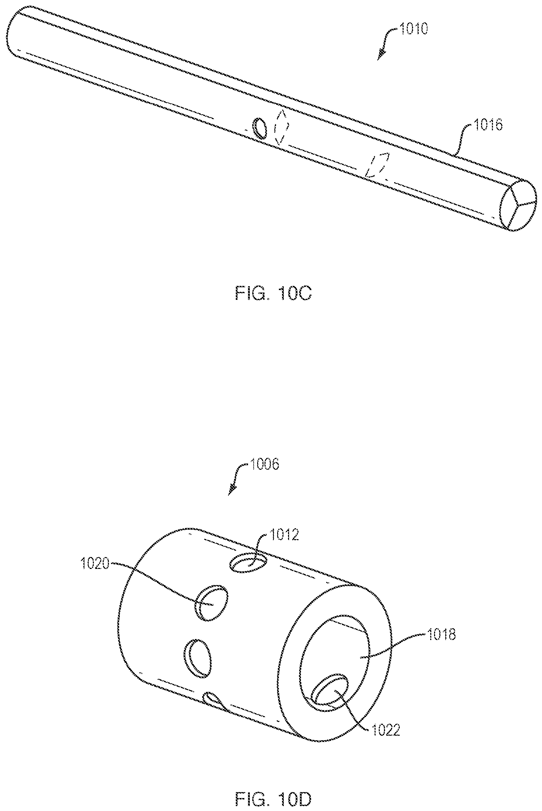

With reference to FIGS. 10A-10D, an embodiment of a device with a flow distribution element includes an elongate member 1010 having a longitudinal axis, an open proximal end, a distal end, and a plurality of lumens 1014 extending therebetween in fluid communication with a flow distribution element. The plurality of lumens 1014 divides the flow of fluid into radial lumens *(i.e.,* rather than one central lumen or multiple lumens). Lumens may have a flow imbalance if the flow is more centralized at the lumens that are most-axial or if the flow is more radial along the outer-most walls of the flow distribution element 1006. A flow distribution element 1006 disposed about the elongate member 1010 includes a plurality of longitudinally adjacent annular chambers 1020, each chamber 1020 having a proximal end, a distal end, and a central lumen 1018 extending therethrough that receives the elongate member 1010. Each chamber 1020 has one or more radial apertures 1012 about an outer surface of the chamber 1020. Each lumen of the plurality of lumens 1014 has at least one supply aperture 1016 in fluid communication with a dedicated chamber for that lumen. A fluid supplied through a device 1000 of this embodiment flows through the elongate member via the three, radially partitioned lumens of the plurality of lumens 1014. The flow within each lumen will exit the lumen through the supply aperture 1016 of the lumen into the dedicated chamber 1006. The flow will then exit the chamber 1006 through the radial apertures 1012 and out of the device 1000. Dividing the flow within the plurality of lumens 1014 to separate chambers 1006 each having their own radial apertures 1012 helps to produce a balanced flow to the radial apertures 1012 and spray from the radial apertures 1012. Each of the plurality of lumens 1014 of the elongate member is dedicated to a respective chamber 1006 and has at least one dedicated supply aperture 1016 in fluid communication therewith. The elongate body 1000 may be mated with a catheter. Each lumen of the plurality of lumens 1014 of the elongate member 1010 may terminate at a supply aperture 1016 associated with its respective annular chamber 1020.

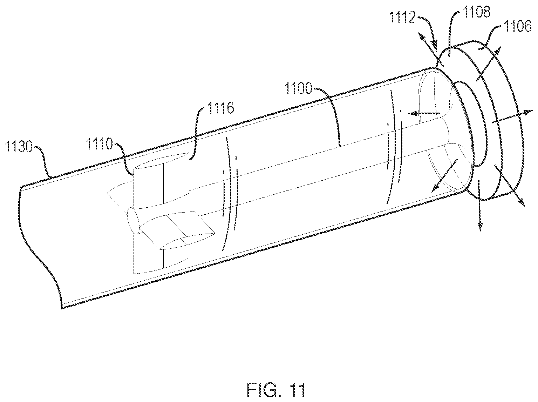

With reference to FIG. 11, an embodiment of a device with a flow distribution element 1110 has the flow distribution element 1110 extending partially along an elongate body 1100. The body 1100 is configured to be inserted into or be integral with a distal end opening of a catheter 1130. The body 1100 has a longitudinal axis, a proximal end, a distal end, and a backstop 1106 at the distal end. The flow distribution element 1110 includes a plurality of fins 1116 extending radially from a longitudinal axis of the elongate body 1100 that are configured to engage an inner surface of the catheter 1130. The backstop 1106 has a surface 1108 perpendicular to and facing the distal end opening of the catheter 1130 when the elongate body 1100 is inserted into the catheter 1130. The surface 1108 is longitudinally offset from a distal end opening of the catheter 1130 in a distal direction forming a radial aperture 1112 around the opening. A 360° aperture 1112 is created by the space between the surface 1108 and the distal end of the catheter 1130. A flow of fluid through the catheter 1130 is directed from a substantially axial direction to a substantially radial direction through the aperture 1112 upon colliding with the surface 1108.

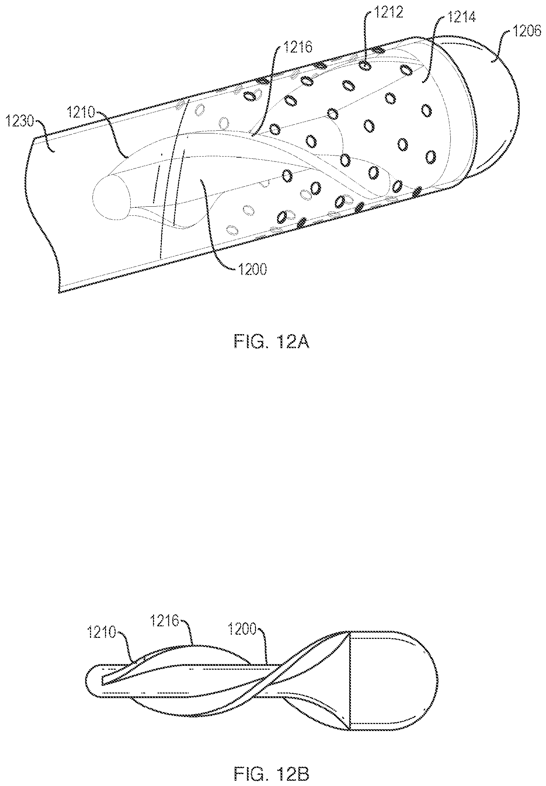

With reference to FIGS. 12A and 12B, an embodiment of a device with a flow distribution element 1210 has the flow distribution element extending partially along an elongate body 1200 and the body 1200 is configured to be inserted into or integral with a catheter 1230. The elongate body 1200 has a proximal end, a distal end, and a backstop 1206 at the distal end. The flow distribution element 1210 includes fins 1216. The fins 1216 gradually extend radially further from a longitudinal axis of the elongate body 1200 as the fins 1216 extend along the elongate body 1200 in the direction of the backstop. The fins 1216 extend along the elongate body 1200 in a helical pattern and in a distal direction. The backstop 1206 is configured to engage the inner surface of the catheter 1230. The elongate body 1200 may engage a catheter 1230 with apertures 1212, which may be radial apertures that receive protrusions on the wall of the catheter 1230. Conversely, the protrusions may be on the device and configured be received within notches on the wall of a catheter 1230. A flow of fluid through the catheter 1230 is directed from a substantially axial direction to a substantially radial direction through the apertures 1212 by the fins 1216 as well as the elongate body 1200 transitioning into the backstop 1206. The elongate body 1200 has a slope 1214 that transitions from the body 1200 to the backstop 1206 such that cross-sectional resistance to the flow of fluid increases as the flow translates distally. The helical pattern of the fins 1216 encourages the flow to rotate, increasing radial flow towards the apertures 1212 due to centripetal acceleration. The slope 1214 portion and fins 1210 of the elongate body 1200 may be independent of the backstop 1206 and instead be disposed on an axial shaft and motor assembly. The motor may rotate the slope 1214 portion and fins 1210, directing the flow of fluid generally toward the apertures 1212.

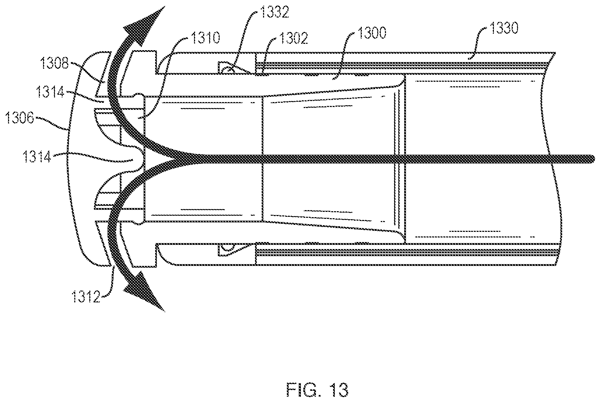

With reference to FIG. 13, an embodiment of a device with a flow distribution element has an elongate body 1300 configured to be inserted into a catheter 1330. The elongate body 1300 engages the catheter 1330 via one or more ridges 1302 along the elongate body 1300. A ring 1332 may fit into a notch of the catheter 1330 such that the body 1300 is held in place. Instead of or in addition to the ring 1332 and/or ridges 1302, the body 1300 may be fixed into place within the catheter 1330 via threading, bonding, interference, and the like. The elongate body 1300 has a proximal end, a distal end, and a backstop 1306 attached at the distal end. The backstop 1306 has a concave surface 1308 facing the distal end opening of the catheter 1330. The flow distribution element 1310 includes a diffuser element 1314 from the concave surface 1308 that extends proximally towards a lumen of the catheter 1330 and against a direction of flow from the opening of the catheter 1330. The diffuser element 1314 has a curved transition from the tip to the concave surface 1308. The backstop 1306 and the flow distribution element 1310 are configured to be concentrically disposed within or integrated as part of a lumen of the elongate body 1300. The concave surface 1308 is offset from the distal end of the elongate body 1300, creating an aperture 1312. The aperture 1312 is oriented 20° proximally from a perpendicular plane to the longitudinal axis of the elongate body 1300. This proximal orientation directs flow from the aperture 1312 proximally such that the fluid from the flow does not spray distally into the body lumen. Other angles of orientation may be selected as desired to achieve a desired deflection pattern. The backstop 1306 may be connected to the elongate body 1300 by several struts of the diffuser element 1314 that engage the elongate body 1300. The struts of the diffuser element 1314 distally span the gap of the aperture 1312, but are thin enough to not substantially obstruct the aperture 1312. The thickness and number of struts in the diffuser element 1314 may be determined by the desired amount of connection strength between the backstop 1306 and the elongate body 1300 and the desired flow of spray from the aperture 1312.

In various embodiments, the apertures may include multiple rows of apertures, such as, e.g., 6 rows spaced about 5 millimeters apart. The apertures may comprise a variety of shapes and sizes, such as, e.g., 24 equally spaced holes of about 0.015" (about 0.381 millimeters) diameter. The apertures may be oriented radially, proximally, or distally, or some combination thereof.

All of the devices and/or methods disclosed and claimed herein can be made and executed without undue experimentation in light of the present disclosure. While the devices and methods of this disclosure have been described in terms of preferred embodiments, it will be apparent to those of skill in the art that variations can be applied to the devices and/or methods and in the steps or in the sequence of steps of the method described herein without departing from the scope of the disclosure. All such similar substitutes and modifications apparent to those skilled in the art are deemed to be within the scope of the disclosure as defined by the appended claims.

## Claims

1. A device for a radial spray catheter (102, 202), comprising:
a body (800) having a longitudinal axis, a proximal end, a distal end, a mid-portion extending therebetween, and an exterior radial surface;
a central lumen extending within the body (800) along the longitudinal axis from the proximal end of the body (800) into at least the mid-portion of the body (800);
one or more apertures (812) distributed about the exterior radial surface of the body (800); and
a flow distribution element in fluid communication with the central lumen and the one or more apertures,
wherein the flow distribution element comprises a distribution lumen (810) that extends from the central lumen to the distal end of the body (800) and is in fluid communication with the central lumen, and wherein the distribution lumen (810) includes a plurality of the apertures (812) along the longitudinal axis,
**characterised by**
a spring (814) within the distribution lumen having a distal component associated with the distal end of the body (800) and a proximal component associated with an oscillator body (816), whereby the oscillator body (816) oscillates in the distribution lumen with flow pushing against a restoring force of the spring (814) to distribute the flow to the apertures (812).

2. The device of claim 1, wherein the central lumen extends through the distal end of the body (800).

3. The device of any of the preceding claims, wherein the proximal end of the body (800) is configured to be mated with a distal end of the catheter (102, 202).

4. The device of any of the preceding claims, wherein the flow distribution element comprises a diffuser element that is coaxial with the central lumen and faces proximally along the longitudinal axis of the body (800).

5. The device of claim 4, wherein the diffuser element is a cone with a cone apex that faces proximally along the longitudinal axis of the body (800).

6. The device of any of claims 1-3 and 5, wherein the flow distribution element comprises a plurality of independent lumens comprising elongate tubes, each tubular lumen associated with an independent aperture, wherein each tubular lumen has a proximal portion that extends distally within the body parallel with the central lumen and along a curve to a radial portion of the tubular lumen that is aligned with the associated aperture.

7. The device of claim 6, wherein the tubular lumens are aligned in concentric radial circles at the proximal portion, and wherein the lumens radially closer to the longitudinal axis of the body (800) extend farther distally at the radial portion than the lumens radially farther from the longitudinal axis of the body (800).

8. The device of any of claims 1-3, 5, and 7, wherein the flow distribution element comprises a distribution lumen that extends from the central lumen to the distal end of the body (800) and is in fluid communication and substantially coaxial with the central lumen, and wherein the distribution lumen has sections in the direction of the distal end along the longitudinal axis of the body that change in inner diameter and each include at least one of the apertures.

## Patentansprüche

1. Vorrichtung für einen Radialspray-Katheter (102, 202), Folgendes umfassend:
einen Körper (800), der eine Längsachse, ein proximales Ende, ein distales Ende, einen sich dazwischen erstreckenden Mittelabschnitt und eine äußere radiale Oberfläche aufweist;
ein zentrales Lumen, das sich innerhalb des Körpers (800) entlang der Längsachse von dem proximalen Ende des Körpers (800) bis mindestens in den Mittelabschnitt des Körpers (800) erstreckt;
eine oder mehrere Öffnungen (812), die um die äußere radiale Oberfläche des Körpers (800) verteilt sind; und
ein mit dem zentralen Lumen und der einen oder den mehreren Öffnungen in Fluidkommunikation stehendes Strömungsverteilungselement,
wobei das Strömungsverteilungselement ein Verteilungslumen (810) umfasst, das sich von dem zentralen Lumen zu dem distalen Ende des Körpers (800) erstreckt und in Fluidkommunikation mit dem zentralen Lumen steht, und wobei das Verteilungslumen (810) eine Vielzahl der Öffnungen (812) entlang der Längsachse beinhaltet,
**gekennzeichnet durch**
eine Feder (814) innerhalb des Verteilungslumens, die eine distale Komponente, die dem distalen Ende des Körpers (800) zugeordnet ist, und eine proximale Komponente, die einem Oszillatorkörper (816) zugeordnet ist, aufweist, wobei der Oszillatorkörper (816) in dem Verteilungslumen oszilliert, wobei eine Strömung gegen eine Rückstellkraft der Feder (814) drückt, um die Strömung zu den Öffnungen (812) zu verteilen.

2. Vorrichtung nach Anspruch 1, wobei sich das zentrale Lumen durch das distale Ende des Körpers (800) erstreckt.

3. Vorrichtung nach einem der vorhergehenden Ansprüche, wobei das proximale Ende des Körpers (800) so konfiguriert ist, dass es mit einem distalen Ende des Katheters (102, 202) zusammenpasst.

4. Vorrichtung nach einem der vorhergehenden Ansprüche, wobei das Strömungsverteilungselement ein Diffusorelement umfasst, das koaxial zu dem zentralen Lumen ist und proximal entlang der Längsachse des Körpers (800) weist.

5. Vorrichtung nach Anspruch 4, wobei das Diffusorelement ein Kegel mit einem Kegelscheitelpunkt ist, der proximal entlang der Längsachse des Körpers (800) weist.

6. Vorrichtung nach einem der Ansprüche 1-3 und 5, wobei das Strömungsverteilungselement eine Vielzahl von unabhängigen Lumen umfasst, die längliche Röhren umfassen, wobei jedes röhrenförmige Lumen einer unabhängigen Öffnung zugeordnet ist, wobei jedes röhrenförmige Lumen einen proximalen Abschnitt aufweist, der sich distal innerhalb des Körpers parallel zu dem zentralen Lumen und entlang einer Krümmung zu einem radialen Abschnitt des röhrenförmigen Lumens erstreckt, das an der zugeordneten Öffnung ausgerichtet ist.

7. Vorrichtung nach Anspruch 6, wobei die röhrenförmigen Lumen an dem proximalen Abschnitt in konzentrischen radialen Kreisen ausgerichtet sind und wobei sich die Lumen radial näher an der Längsachse des Körpers (800) weiter distal an dem radialen Abschnitt erstrecken als die Lumen radial weiter von der Längsachse des Körpers (800).

8. Vorrichtung nach einem der Ansprüche 1-3, 5 und 7, wobei das Strömungsverteilungselement ein Verteilungslumen umfasst, das sich von dem zentralen Lumen zu dem distalen Ende des Körpers (800) erstreckt und in Fluidkommunikation mit dem zentralen Lumen steht und im Wesentlichen koaxial dazu ist, und wobei das Verteilungslumen Bereiche in der Richtung des distalen Endes entlang der Längsachse des Körpers aufweist, die sich im Innendurchmesser ändern und jeweils mindestens eine der Öffnungen beinhalten.

## Revendications

1. Dispositif pour un cathéter à pulvérisation radiale (102, 202), comprenant :
un corps (800) ayant un axe longitudinal, une extrémité proximale, une extrémité distale, une partie médiane se prolongeant entre ceux-ci, et une surface radiale extérieure ;
une lumière centrale se prolongeant à l'intérieur du corps (800) le long de l'axe longitudinal depuis l'extrémité proximale du corps (800) jusque dans au moins la partie médiane du corps (800) ;
une ou plusieurs ouvertures (812) distribuées autour de la surface radiale extérieure du corps (800) ; et
un élément de distribution d'écoulement en communication fluidique avec la lumière centrale et les une ou plusieurs ouvertures,
dans lequel l'élément de distribution d'écoulement comprend une lumière de distribution (810) qui se prolonge de la lumière centrale à l'extrémité distale du corps (800) et est en communication fluidique avec la lumière centrale, et dans lequel la lumière de distribution (810) comporte une pluralité d'ouvertures (812) le long de l'axe longitudinal,
**caractérisé par**
un ressort (814) à l'intérieur de la lumière de distribution ayant un composant distal associé à l'extrémité distale du corps (800) et un composant proximal associé à un corps d'oscillateur (816), moyennant quoi le corps d'oscillateur (816) oscille dans la lumière de distribution avec un écoulement poussant contre une force de rappel du ressort (814) pour distribuer l'écoulement vers les ouvertures (812).

2. Dispositif selon la revendication 1, dans lequel la lumière centrale se prolonge à travers l'extrémité distale du corps (800).

3. Dispositif selon l'une quelconque des revendications précédentes, dans lequel l'extrémité proximale du corps (800) est configurée pour être accouplée à une extrémité distale du cathéter (102, 202).

4. Dispositif selon l'une quelconque des revendications précédentes, dans lequel l'élément de distribution d'écoulement comprend un élément diffuseur qui est coaxial par rapport à la lumière centrale et fait face de manière proximale le long de l'axe longitudinal du corps (800).

5. Dispositif selon la revendication 4, dans lequel l'élément diffuseur est un cône muni d'un sommet de cône qui fait face de manière proximale le long de l'axe longitudinal du corps (800).

6. Dispositif selon l'une quelconque des revendications 1 à 3 et 5, dans lequel l'élément de distribution d'écoulement comprend une pluralité de lumières indépendantes comprenant des tubes allongés, chaque lumière tubulaire étant associée à une ouverture indépendante, dans lequel chaque lumière tubulaire a une partie proximale qui se prolonge distalement à l'intérieur du corps parallèlement à la lumière centrale et le long d'une courbe jusqu'à une partie radiale de la lumière tubulaire qui est alignée avec l'ouverture associée.

7. Dispositif selon la revendication 6, dans lequel les lumières tubulaires sont alignées en cercles radiaux concentriques au niveau de la partie proximale, et dans lequel les lumières radialement plus proches de l'axe longitudinal du corps (800) se prolongent plus loin distalement au niveau de la partie radiale que les lumières radialement plus éloignées de l'axe longitudinal du corps (800).

8. Dispositif selon l'une quelconque des revendications 1 à 3, 5 et 7, dans lequel l'élément de distribution d'écoulement comprend une lumière de distribution qui se prolonge de la lumière centrale à l'extrémité distale du corps (800) et est en communication fluidique et sensiblement coaxiale par rapport à la lumière centrale, et dans lequel la lumière de distribution a des sections dans la direction de l'extrémité distale le long de l'axe longitudinal du corps qui changent de diamètre interne et comprennent chacune au moins une des ouvertures.
